(19) Europäisches Patentamt
European Patent Office
Office européen des brevets

(11) **EP 4 004 169 B1**

(12) **EUROPEAN PATENT SPECIFICATION**

(45) Date of publication and mention
of the grant of the patent:
**19.04.2023   Bulletin 2023/16**

(21) Application number: **20746211.0**

(22) Date of filing: **27.07.2020**

(51) International Patent Classification (IPC):
*C11D 3/48* (2006.01)      *C11D 17/06* (2006.01)
*A61K 8/49* (2006.01)      *A61Q 5/00* (2006.01)
*A61K 8/02* (2006.01)

(52) Cooperative Patent Classification (CPC):
**C11D 3/48; A61K 8/0225; A61K 8/4926;
A61Q 5/006; C11D 17/06**

(86) International application number:
**PCT/EP2020/071162**

(87) International publication number:
**WO 2021/018853 (04.02.2021 Gazette 2021/05)**

(54) **PROCESS FOR MAKING PIROCTONE OLAMINE GRANULES**

VERFAHREN ZUR HERSTELLUNG VON PIROCTONOLAMINGRANULATEN

PROCÉDÉ DE FABRICATION DE GRANULES DE PIROCTONE OLAMINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.07.2019   EP 19189413**

(43) Date of publication of application:
**01.06.2022   Bulletin 2022/22**

(73) Proprietor: **Clariant International Ltd
4132 Muttenz (CH)**

(72) Inventors:
• **DYBALLA, Michael**
**60437 Frankfurt (DE)**
• **BRAUN, Lisa**
**65779 Kelkheim (Taunus) (DE)**
• **GROESCHEN, Mathias**
**65620 Waldbrunn-Hintermeilingen (DE)**
• **KLUG, Peter**
**63762 Großostheim (DE)**

(74) Representative: **Kampen, Daniela
Clariant Produkte (Deutschland) GmbH
Patent & License Management Chemicals
Industriepark Höchst / G 860
65926 Frankfurt am Main (DE)**

(56) References cited:
**US-A- 4 796 812**

• **KLEINEBUDDE P ED - LENDLEIN ANDREAS ET
AL: "Roll compaction/dry granulation:
pharmaceutical applications", EUROPEAN
JOURNAL OF PHARMACEUTICS AND
BIOPHARMACEUTICS, ELSEVIER SCIENCE
PUBLISHERS B.V., AMSTERDAM, NL, vol. 58, no.
2, 1 September 2004 (2004-09-01), pages 317-326,
XP004526315, ISSN: 0939-6411, DOI:
10.1016/J.EJPB.2004.04.014**

**Description**

The present invention relates to a process for providing piroctone olamine granules having a target particle size range and to piroctone olamine granules having a target particle size range.

With reference to DE 2 234 009 A1 and DE 1 795 270 A1, 1-hydroxy-4-methyl-6-(2,4,4-trimethyl)-pentyl-2(1H)-pyridone, 2-aminoethanol salt, also known as piroctone ethanolamine or piroctone olamine, is an anti-fungal active agent which is effective against the causes of dandruff. It is known to include piroctone olamine in personal care products, such as shampoos. DE 1 795 270 A1 also describes a method of making piroctone olamine. US4796812 A discloses powdered piroctone olamine granules.

Piroctone olamine exists in the form of crystals which may be added to personal care products. Commercially available piroctone olamine made by processes such as those referred to above, typically has primary crystals with a diameter/length (D/l) ratio of about 1:7 and a median diameter ($d_{50}$) in the region of about 100 micrometers. The primary crystals are those formed during the crystallization process, but prior to further processing and bulk handling steps. After such further processing and handling in bulk quantities, the crystals may change dimensions. In particular, the primary crystals may fracture and break, such that $d_{50}$ for such bulk quantities may be smaller than $d_{50}$ for primary crystals. Such bulk quantities of piroctone olamine crystals may gather together to form clumps, especially during storage. Clumping phenomena may give rise to difficulties when handling and processing the bulk crystals.

It is with this background that the present invention has been devised.

According to a first aspect, the invention relates to a process according to claim 1, for providing piroctone olamine granules having a target particle size range, comprising:

a) Compressing piroctone olamine crystals to form a compactate;
b) Milling the compactate to form bulk granules of piroctone olamine.

Advantageously, the process further comprises:

c) Separating piroctone olamine granules having the target particle size range from the bulk granules.

Without wishing to be bound by theory, it is considered that certain particle size ranges, especially certain ranges comprising particle sizes which are larger than the primary crystals, may remain more flowable during storage and be less liable to clumping.

The compression in a) may be performed in the absence or presence of additives. According to one embodiment, pure piroctone olamine crystals, comprising no additives or other materials, are compressed in a). In one embodiment, the compression in a) is performed in the absence of any additives. In one embodiment, the compression in a) is performed in the absence of any plastifiers and lubricants. According to another embodiment, the piroctone olamine crystals are mixed with an additive, such as a plastifier or a lubricant. Suitable additives which may be employed in this instance include polyethylene glycol, stearic acid or ethylene glycol distearate. The compression in a) may be performed in the absence or presence of water. In the compression in a), the amount of water is typically less than 3%, preferably less than 2%, more preferably less than 1%, particularly preferably less than 0.5%.

According to one embodiment of the first aspect of the invention, the compression in a) is performed at a pressure from 25 bar to 200 bar and preferably at a pressure from 30 bar to 50 bar.

According to another embodiment of the first aspect of the invention, the pressure applied in compression step a) is sufficient to form a compactate having a density from 920kg/m$^3$ to 1300kg/m$^3$. For completeness, this is the actual density of the compactate, not a bulk density.

The term "compactate" as used herein is well known to a person skilled in the art. The compactate can be in any form. For example, the compactate can be in the form of briquettes, cigars, tablets or "Schülpen". Preferably, the compactate is in the form of briquettes or "Schülpen". Particularly preferably, the compactate is in the form of briquettes. Also particularly preferably, the compactate is in the form of "Schülpen". According to another embodiment of the first aspect of the invention, in b) milling takes place in a sieve mill, such as a rotary sieve mill or an oscillating sieve mill. In such a case, the mesh of the sieve mill may have a mesh size of 10mm or less, preferably a mesh size of 7mm or less, more preferably a mesh size of 4mm or less, more preferably still a mesh size of 2mm. It is not essential to use a sieve mill, however, and a skilled person would be able to select alternative milling devices.

The target particle size range may be determined according to an appropriate metric selected by the skilled person. According to one embodiment, the metric is a target $d_{50}$ range. The target $d_{50}$ range is from 0.3mm to 6mm, preferably from 0.3mm to 2.5mm, more preferably still 0.5mm to 2mm.

Advantageously, according to the first aspect of the invention, the process further comprises:
c) Separating piroctone olamine granules having the target particle size range from the bulk granules.

In one embodiment, in c) separating piroctone olamine granules in the target particle size range comprises:

c1) An optional first separation performed on the bulk granules using a first sieve having a mesh size corresponding to the upper limit of the target particle size range, wherein the first separation yields an intermediate product comprising piroctone olamine granules which have passed through the first sieve and a remnant comprising coarse piroctone olamine granules which have not;

c2) A second separation performed on the intermediate product or, if there is no first separation, then on the bulk granules, using a second sieve having a mesh size corresponding to the lower limit of the target particle size range, wherein the second separation yields piroctone olamine fines which have passed through the second sieve and piroctone olamine granules in the target particle size range which have not.

According to another embodiment, the first separation is performed with a vibrating sieve or an air jet sieve and/or the second separation is performed with a vibrating sieve or an air jet sieve. For the event that milling in b) takes place in a mill, such as a sieve mill, that allows granules below a certain size to be generated with a high degree of accuracy, then the first separation may not be necessary, because it may effectively be carried out in the mill. Even then, however, it may still be desirable to perform a first separation, because a small proportion of the granules which are larger than the upper limit of the target particle size range, for example if they are shaped as needles, may pass through a mill, such as a sieve mill.

Advantageously, according to the first aspect of the invention, the process further comprises:

d) Recirculating the piroctone olamine fines and adding them to the piroctone olamine crystals to be compressed in a).

Advantageously, according to the first aspect of the invention, for the case in which a first separation has taken place, the process further comprises:

e) Milling the remnant;

f) Recirculating the milled remnant and adding it to the piroctone olamine crystals to be compressed in a).

Performing one or both of these recirculation steps, while not essential for production of the granules of the final product, is observed to provide a more compact compactate and therefore more compact granules. This is at least partially due to the fact that the recirculated product has been pre-compacted. Recirculation improves the yield of piroctone olamine granules having the target particle size range, not only because recirculation wastes less product, but also because the more compact compactate (and granules) are less friable.

According to a second aspect of the invention, piroctone olamine granules are provided having $d_{50}$, based on volume distribution, from 0.3mm to 6mm, preferably from 0.3mm to 2.5mm, more preferably still 0.5mm to 2mm, and having an average D/l ratio, of diameter (D) to length (l) from 0.6 to 1.0, preferably from 0.7 to 1.0, particularly preferably from 0.8 to 1.0. Without wishing to be bound by theory, it is considered that more "square" or cubic particles may remain more flowable during storage and be less liable to clumping.

According to another embodiment, the piroctone olamine granules have a $d_{90}$ of less than or equal to 1.9mm.

According to a further embodiment, the piroctone olamine granules have a $d_{10}$ of greater than or equal to 0.4mm.

According to another embodiment, the piroctone olamine granules have a bulk density from 400kg/m$^3$ to 600kg/m$^3$, preferably from 450kg/m$^3$ to 550kg/m$^3$, more preferably from 470kg/m$^3$ to 530kg/m$^3$.

In this document, including in all embodiments of all aspects of the present invention, the following definitions apply unless specifically stated otherwise.

In relation to the particle size distribution measures used herein, $d_{50}$, d(50) or D50, the median, is defined as the diameter where half of the population lies below this value. Similarly, 10 percent of the population lies below the $d_{10}$, d(10) or D10 diameter and 90 percent of the population lies below the $d_{90}$, d(90) or D90 diameter. If not stated otherwise, the $d_{50}$, $d_{10}$, $d_{90}$ values are based on a volume distribution.

All percentages are by weight (w/w) of the total composition. All ratios are weight ratios. "wt.%" means percentage by weight. References to 'parts' e.g. a mixture of 1 part X and 3 parts Y, is a ratio by weight. "QS" or "QSP" means sufficient quantity for 100% or for 100g. +/- indicates the standard deviation. All ranges are inclusive and combinable. The number of significant digits conveys neither a limitation on the indicated amounts nor on the accuracy of the measurements. All measurements are understood to be made at 23°C and at ambient conditions, where "ambient conditions" means at 1 atmosphere (atm) of pressure and at 50% relative humidity. "Relative humidity" refers to the ratio (stated as a percent) of the moisture content of air compared to the saturated moisture level at the same temperature and pressure. Relative humidity can be measured with a hygrometer, in particular with a probe hygrometer from VWR® International. Herein "min" means "minute" or "minutes". Herein "mol" means mole. Herein "g" following a number means "gram" or "grams" and "kg" means "kilogram" or "kilograms". Herein, "comprising" means that other steps and other ingredients can be in addition. Embodiments and aspects described herein may comprise or be combinable with elements, features or components of other embodiments and/or aspects despite not being expressly exemplified in combination, unless an incompatibility is stated. "In at least one embodiment" means that one or more embodiments, optionally all embodiments or a large subset of embodiments, of the present invention has/have the subsequently described feature. "Molecular weight" or "M.Wt." or "MW" and grammatical equivalents mean the number average molecular weight.

The invention will now be further described with reference to the accompanying drawings, in which:

Figure 1 illustrates the volumetric distribution density of particles, q (left hand y-axis) and the volumetric cumulative distribution Q(r) (right hand y-axis) versus diameter (x-axis) of crystals of piroctone olamine which have not been subjected to the process according to the invention.

Figure 2 illustrates the volumetric distribution density of particles, q (left hand y-axis) and the volumetric cumulative distribution Q(r) (right hand y-axis) versus diameter (x-axis) of granules of piroctone olamine which have been subjected to the process according to the invention.

Figure 3 illustrates a microscope image of some piroctone olamine granules according to the invention. The image is divided into 4 parts using a reticle scale in preparation for making width and length measurements in the fashion described below.

Particle Size Distribution (PSD) Measurement Method for Piroctone Olamine Crystals

For measuring the PSD of the piroctone olamine crystals (the starting material), a Horiba LA-950 particle size analyzer was used for measuring the diameter, the volumetric distribution density and the volumetric cumulative distribution of the crystals. The analyzer uses a laser diffraction method (ISO 13320:2009, Fraunhofer Diffraction Method) to measure the distribution and is based on the direct proportionality of the intensity of light scattered by a particle, to the diameter. Furthermore the scattering angle is inversely proportional to the diameter and vice versa.

In preparation for the analysis, the required amount of crystals is placed on a sieve with a mesh size of 1 mm. The crystals are sieved with an amplitude of 1.5 mm for 3 minutes.

The required amount of sieved crystals was added to the gutter of the dry dispersion unit.

Three measurements were made in the HORIBA LA-950 particle size analyzer with the following parameters:

- Gutter starting value 85 -110 (unit-less), automatic control
- Dispersing pressure 0.3 MPa

The three measurements are combined with the software to form an averaged measurement. For analysis the focus is on $d_{10}$, $d_{50}$ and $d_{90}$ volume fractions.

Particle Size Distribution Measurement Method of Piroctone Olamine Granules

For piroctone olamine granules, which have been compressed according to the invention, a Retsch Sieve Maschine "AS200 Control" was used for measuring the diameter, the volumetric distribution density and the volumetric cumulative distribuition of the granules. This is referred to herein as a sieve analysis.

In preparation for the sieve analysis, sieves are stacked one above the other (sieve tower) and fixed in the sieve machine. The mesh sizes of sieves in the sieve tower increase in size from the bottom to the top of the tower. Analytical sieves (DIN ISO 3310-1) with dimensions of 200x50 mm are used.

An appropriate quantity of granules (80-120g) is placed on the sieve with the largest mesh size (at the top of the sieve tower) and the granules are sieved with an amplitude of 1 mm for 2 minutes.

By weighing the product on every single sieve, a particle size distribution is calculated with the Retsch software ("EasySieve").

Measurement of the Width/Length Ratio of the Piroctone Olamine Crystals

A small amount of sieved crystals (see above) is spread on a Petri dish with a spatula.

Under a microscope, a position is sought in which isolated crystals are clearly visible. The microscope used was a Keyence VHX 2000 series digital micoscope with a VH-Z20W zoom lens, using a VHX-S90BE free angle observation system. The microscope does not form part of the invention and a skilled person could select suitable alternative microscopes.

The limits are set for the depth of field and an image in the appropriate magnification with the depth of field function of the microscope made.

Pictures are taken at the following magnifications x50, x100, 150, x200 in order to obtain an overall impression of the bulk crystals.

A position on the Petri dish is needed in which an area of 3×3 images can be made with as many individual crystals as possible.

A 3x3 merged image (that is nine images, merged into one) is created at a magnification of x200.

The image is divided into 4 parts using a reticle scale. In each quarter 5 representative crystals are selected (20 crystals in total). For each of these 20 crystals, the diameter (D) and length (l) and the D/l ratio are determined. The average D/l ratio for all the crystals is then calculated, which is the sum of the measured D/l divided by the number of crystals (ΣD/l)/20).

Measurement of the Width/Length Ratio of the Piroctone Olamine Granules

A small amount of sieved granules (see above) is spread on a Petri dish with a spatula.

Under a microscope, a position is sought in which isolated granules are clearly visible. The microscope used was a Keyence VHX 2000 series digital micoscope with a VH-Z20W zoom lens, using a VHX-S90BE free angle observation system. The microscope does not form part of the invention and a skilled person could select suitable alternative microscopes.

The limits are set for the depth of field and an image in the appropriate magnification with the depth of field function of the microscope made.

A position on the Petri dish is needed in which an area of 3×3 images can be made with as many individual granules as possible.

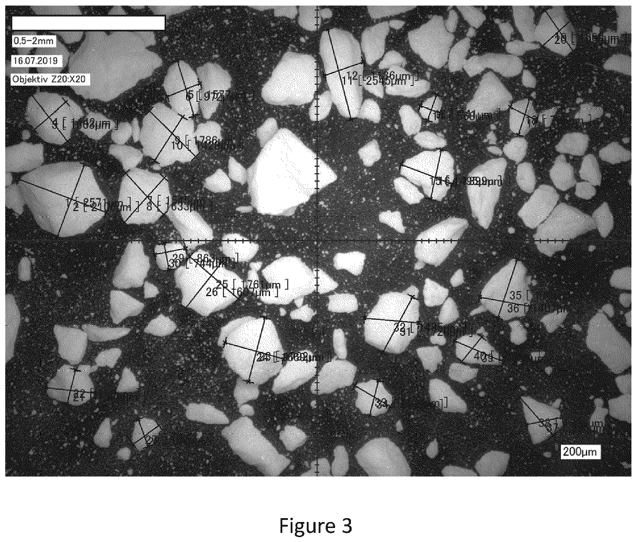

A 3×3 merged image (that is, nine images merged into one) is created at a magnification of x20. Figure 3 illustrates such an image.

The image is divided into 4 parts using a reticle scale. In each quarter 5 representative granules are selected (20 granules in total). For each of these 20 granules, the diameter (D) and length (l) and the D/l ratio are determined. The average D/l ratio for all the granules is then calculated, which is the sum of the measured D/l divided by the number of granules (ΣD/l)/20).

Flowability Measurement

Clumping of the crystals or granules may be regarded as a low degree of flowability. In order to obtain an objective measurement of clumping, therefore, the crystals' / granules' flowability may be measured. The skilled person would be aware of other ways to characterize clumping.

The flowability of a bulk solid may be characterized by its unconfined yield strength, $\sigma_c$, in dependence on consolidation stress, $\sigma_1$, and storage period, t. Usually the ratio $ff_c$ of consolidation stress, $\sigma_1$, to unconfined yield strength, $\sigma_c$, is used to characterize flowability numerically:

$$ff_c = \sigma_1 / \sigma_c$$

The larger $ff_c$ is, i.e., the smaller the ratio of the unconfined yield strength, $\sigma_c$, to the consolidation stress, $\sigma_1$, the better a bulk solid flows. Flow behavior is defined as follows:

$ff_c$ of less than 1, not flowing
$ff_c$ from 1 to less than 2, very cohesive
$ff_c$ from 2 to less than 4, cohesive
$ff_c$ from 4 to less than 10, easy flowing
$ff_c$ of greater than 10, free flowing

The parameter $ff_c$ may be generated using a ring sheer test in the fashion described by Schulze, D (2009) "Pulver und Schüttgüter", 2nd Edition, Springer, Berlin. This method does not form part of the present invention and is merely referred to as one way to characterize flowability in order to demonstrate the effect of the more flowable nature of the granules according to the invention versus piroctone olamine crystals. The skilled person would be aware of other ways to characterize flowability.

Example 1

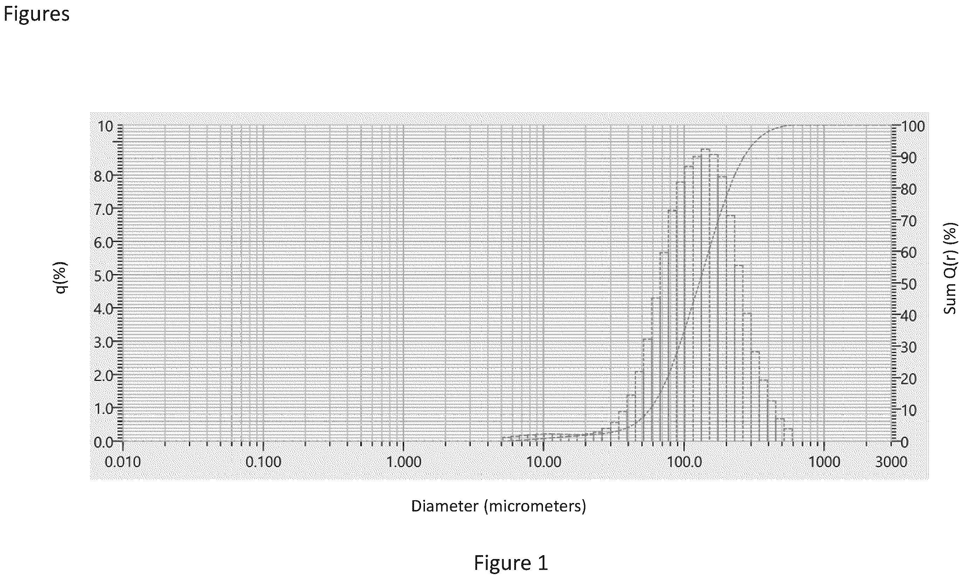

The starting material comprised 65% piroctone olamine crystals having the particle size distribution characteristics given in Table 1 and 35% of recycled fines (granules with $d_{50}$ of 0-0.5mm). The recycled fines consist of 100% compacted piroctone olamine from the previous run. Figure 1 illustrates the volumetric distribution density of particles, q (left hand y-axis) and the volumetric cumulative distribution Q(r) (right hand y-axis) versus diameter (x-axis) of crystals of piroctone olamine starting material.

| Table 1 | |
|---|---|
| | |
| $d_{10}$ | 0.06 mm |
| $d_{50}$ | 0.13 mm |
| $d_{90}$ | 0.27 mm |
| **Average D/l** | 0.29 |

The devices used were as follows:
Compression Step a) was performed with an Hosokawa-Alpine "Pharmapaktor L200/50P" compactor having a concave plain roller diameter of 200mm and width of 50mm and a maximal compression force of 150kN.

Milling Step b) was performed using with an Hosokawa-Alpine "FlakeCrusher FC200" sieve mill having a rotor diameter of 150mm and a rotor length of 200mm and a sieve mesh size of 2mm.

Separating Step c) was performed using an Allgaier Vibrating Tumbler Screening Machine VTS 800 comprising a sieve of 800mm diameter with a 0.50mm mesh size.

Important parameters of the process in this example are given in Table 2. Piroctone olamine granules having a target particle size range is referred to below as "final product".

| Table 2 | | |
|---|---|---|
| Recycled fines from a previous run | %wt | 35 |
| Fresh piroctone olamine crystals having the properties given in Table 1 | %wt | 65 |
| Bulk density of the starting material | kg/m³ | 464 |
| Rollers | | 2 concave plain rollers |
| Roller diameter | Mm | 200 |
| Working width of roller | Mm | 50 |
| Roller gap | Mm | 5 |
| Geometry of the feeding screw | | 60/66/120 |
| Target compression force | kN | 50 |
| Specific compression force | N/mm² | 5 |
| Roller rpm | Min⁻¹ | 12 |
| Feeding screw rpm | Min⁻¹ | 34 |
| Product temperature | °C | 38 |
| Compactor throughput | kg/h | 101 |
| Sieve mill FC200 throughput | kg/h | 250 |
| Compactate density | kg/m³ | 1030 |
| Compactate thickness | Mm | 5 |
| Final product bulk density | kg/m³ | 528 |
| Final product yield | %wt | 65% |
| Fines density ($d_{50}$ of 0-0.5mm) | kg/m³ | 460 |

The final product was analysed and had the properties given in Table 3. The image in Figure 3 was used to determine the average D/l value in the fashion described above.

| Table 3 | |
| --- | --- |
| | |
| $d_{10}$ | 0.5 mm |
| $d_{50}$ | 1.1 mm |
| $d_{90}$ | 1.8 mm |
| Average D/l | 0.84 |

For completeness, if the above process is performed in exactly the same way, but without recirculation of fines, then the final product bulk density is found to be $499 kg/m^3$, which is below the value of $528 kg/m^3$ measured (see Table 2) when fines are recirculated. A value of $499 kg/m^3$ is an acceptable final product bulk density, but the higher densities achieved via recirculation are preferred, because of the improved yield, as explained above.

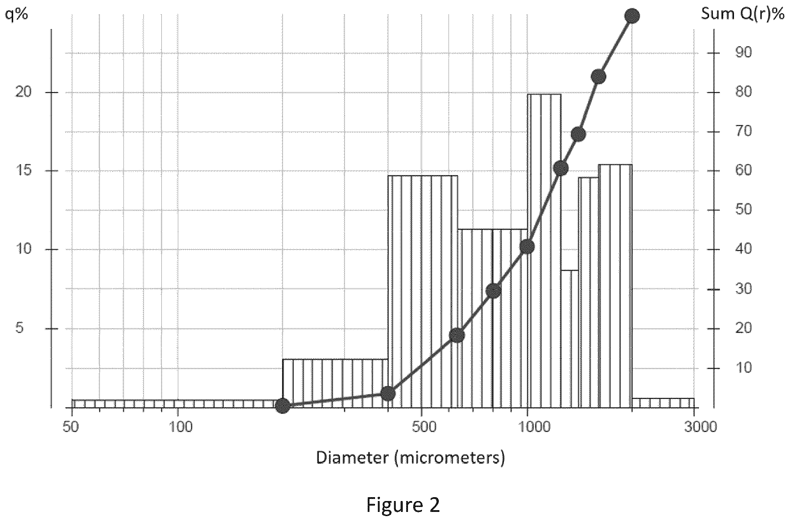

Figure 2 illustrates the volumetric distribution density of particles, q (left hand y-axis) and the volumetric cumulative distribution Q(r) (right hand y-axis) versus diameter (x-axis) of granules of piroctone olamine final product.

A flowability comparison between the starting material and the final product is provided in Table 4. In all cases, storage was at 35 degrees Celsius and 0% relative humidity under consolidation via application of a 2kPa vertical pressure.

| Table 4 | | |
| --- | --- | --- |
| | Flowability Factor, $ff_c$, measured after 20 hours | Flowability Factor, $ff_c$, measured after 168 hours |
| Starting material (piroctone olamine crystals having the properties in Table 1) | 2.1, cohesive | N/A |
| Final product (piroctone olamine granules having the properties in Table 3) | 19.9, free flowing | 11.4, free flowing |

The results demonstrate that, under identical storage and consolidation conditions, piroctone olamine granules having the target particle size are significantly more flowable and therefore less liable to clumping than the non-recrystallized product.

## Claims

1. A process for providing piroctone olamine granules having a target particle size range, wherein the target particle size range is a target $d_{50}$ range, wherein the target $d_{50}$ range, based on a volume distribution, is from 0.3mm to 6mm, and wherein the piroctone olamine granules have an average D/l ratio, of diameter (D) to length (l) from 0.6 to 1.0, comprising:

   a) Compressing piroctone olamine crystals to form a compactate;
   b) Milling the compactate to form bulk granules of piroctone olamine.

2. The process of claim 1, further comprising:
   c) Separating piroctone olamine granules having the target particle size range from the bulk granules.

3. The process of claim 1 or 2, wherein the compression in a) is performed at a pressure from 25 bar to 200 bar, preferably from 30 bar to 50 bar.

4. The process of any preceding claim, wherein the pressure applied in compression step a) is sufficient to form a compactate having a density from $980 kg/m^3$ to $1300 kg/m^3$.

5. The process of any preceding claim, wherein in b) milling takes place in a sieve mill, preferably a rotary sieve mill or an oscillating sieve mill.

**6.** The process of claim 5, wherein the mesh of the rotary sieve mill or the oscillating sieve mill has a mesh size of 10mm or less, preferably a mesh size of 7mm or less, more preferably a mesh size of 4mm or less, more preferably still a mesh size of 2mm.

**7.** The process of any preceding claim, wherein the target $d_{50}$ range, based on a volume distribution, is from 0.3mm to 2.5mm, more preferably still 0.5mm to 2mm.

**8.** The process of any of claims 2 to 7, wherein in c) separating piroctone olamine granules in the target particle size range comprises:

c1) An optional first separation performed on the bulk granules using a first sieve having a mesh size corresponding to the upper limit of the target particle size range, wherein the first separation yields an intermediate product comprising piroctone olamine granules which have passed through the first sieve and a remnant comprising coarse piroctone olamine granules which have not;
c2) A second separation performed on the intermediate product or, if there is no first separation, then on the bulk granules, using a second sieve having a mesh size corresponding to the lower limit of the target particle size range, wherein the second separation yields piroctone olamine fines which have passed through the second sieve and piroctone olamine granules in the target particle size range which have not.

**9.** The process of claim 8, further comprising:
d) Recirculating the piroctone olamine fines and adding them to the piroctone olamine crystals to be compressed in a).

**10.** The process of claim 8 or 9, wherein a first separation has taken place, further comprising:

e) Milling the remnant;
f) Recirculating the milled remnant and adding it to the piroctone olamine crystals to be compressed in a).

**11.** The process of any of the preceding claims, wherein the compression in a) is performed in the absence of any additives.

**12.** The process of any of the preceding claims, wherein the piroctone olamine granules have an average D/l ratio, of diameter (D) to length (l) from 0.7 to 1.0, more preferably from 0.8 to 1.0.

**13.** Piroctone olamine granules having $d_{50}$, based on a volume distribution, from 0.3mm to 6mm, and having an average D/l ratio, of diameter (D) to length (l) from 0.6 to 1.0.

**14.** The piroctone olamine granules of claim 13 having $d_{50}$, based on a volume distribution, from 0.3mm to 2.5mm, more preferably still 0.5mm to 2mm.

**15.** The piroctone olamine granules of claim 13 or 14 having an average D/l ratio, of diameter (D) to length (l) from 0.7 to 1.0, more preferably from 0.8 to 1.0.

**16.** The piroctone olamine granules of any of claims 13 to 15 having a $d_{90}$, based on a volume distribution, of less than or equal to 1.9mm.

**17.** The piroctone olamine granules of any of claims 13 to 16 having a $d_{10}$, based on a volume distribution, of greater than or equal to 0.4mm.

**18.** The piroctone olamine granules of any of claims 13 to 17 having a bulk density from $400kg/m^3$ to $600kg/m^3$, preferably from $450kg/m^3$ to $550kg/m^3$, more preferably from $470kg/m^3$ to $530kg/m^3$.


**Patentansprüche**

**1.** Verfahren zur Bereitstellung von Piroctone-Olamine-Granulat mit einem Zielteilchengrößenbereich, wobei es sich bei dem Zielteilchengrößenbereich um einen Ziel-$d_{50}$-Bereich handelt, wobei der Ziel-$d_{50}$-Bereich, bezogen auf eine Volumenverteilung, 0,3 mm bis 6 mm beträgt und wobei das Piroctone-Olamine-Granulat ein durchschnittliches D/l-Verhältnis von Durchmesser (D) zu Lange (1) von 0,6 bis 1,0 aufweist, umfassend:

a) Komprimieren von Piroctone-Olamine-Kristallen unter Bildung eines Kompaktats;
b) Mahlen des Kompaktats unter Bildung von Bulkgranulat von Piroctone Olamine.

2. Verfahren nach Anspruch 1, ferner umfassend:
c) Abtrennen von Piroctone-Olamine-Granulat mit dem Zielteilchengrößenbereich aus dem Bulkgranulat.

3. Verfahren nach Anspruch 1 oder 2, wobei die Kompression in a) bei einem Druck von 25 bar bis 200 bar, vorzugsweise von 30 bar bis 50 bar, durchgeführt wird.

4. Verfahren nach einem der vorhergehenden Ansprüche, wobei der im Kompressionsschritt a) angewendete Druck zur Bildung eines Kompaktats mit einer Dichte von 980 kg/m$^3$ bis 1300 kg/m$^3$ ausreicht.

5. Verfahren nach einem der vorhergehenden Ansprüche, wobei in b) das Mahlen in einer Siebmühle, vorzugsweise einer Rotorsiebmühle oder einer Schwingsiebmühle erfolgt.

6. Verfahren nach Anspruch 5, wobei die Maschen der Rotorsiebmühle oder Schwingsiebmühle eine Maschenweite von 10 mm oder weniger, vorzugsweise eine Maschenweite von 7 mm oder weniger, weiter bevorzugt eine Maschenweite von 4 mm oder weniger, noch weiter bevorzugt eine Maschenweite von 2 mm, aufweisen.

7. Verfahren nach einem der vorhergehenden Ansprüche, wobei der Ziel-$d_{50}$-Bereich, bezogen auf eine Volumenverteilung, 0,3 mm bis 2,5 mm, noch weiter bevorzugt 0,5 mm bis 2 mm, beträgt.

8. Verfahren nach einem der Ansprüche 2 bis 7, wobei in c) das Abtrennen von Piroctone-Olamine-Granulat in dem Zielteilchengrößenbereich Folgendes umfasst:

c1) eine fakultative erste Trennung, die an dem Bulkgraluat durchgeführt wird, unter Verwendung eines ersten Siebs mit einer Maschenweite, die der Obergrenze des Zielteilchengrößenbereichs entspricht, wobei die erste Trennung ein Zwischenprodukt, das Piroctone-Olamine-Granulat umfasst, das durch das erste Sieb hindurchgegangen ist, und einen Rest, der grobes Piroctone-Olamine-Granulat umfasst, bei dem dies nicht der Fall ist, liefert;
c2) eine zweite Trennung, die an dem Zwischenprodukt oder im Fall der Abwesenheit einer ersten Trennung an dem Bulkgraluat durchgeführt wird, unter Verwendung eines zweiten Siebs mit einer Maschenweite, die der Untergrenze des Zielteilchengrößenbereichs entspricht, wobei die zweite Trennung Piroctone-Olamine-Feingut, das durch das zweite Sieb hindurchgegangen ist, und Piroctone-Olamine-Granulat im Zielteilchengrößenbereich, bei dem dies nicht der Fall ist, liefert.

9. Verfahren nach Anspruch 8, ferner umfassend:
d) Rezirkulieren des Piroctone-Olamine-Feinguts und Zugeben des Feinguts zu den in a) zu komprimierenden Piroctone-Olamine-Kristallen.

10. Verfahren nach Anspruch 8 oder 9, wobei eine erste Trennung erfolgt ist, die ferner Folgendes umfasst:

e) Mahlen des Rests;
f) Rezirkulieren des gemahlenen Rests und Zugeben des Rests zu den in a) zu komprimierenden Piroctone-Olamine-Kristallen.

11. Verfahren nach einem der vorhergehenden Ansprüche, wobei die Kompression in a) in Abwesenheit jeglicher Additive durchgeführt wird.

12. Verfahren nach einem der vorhergehenden Ansprüche, wobei das Piroctone-Olamine-Granulat ein durchschnittliches D/l-Verhältnis von Durchmesser (D) zu Länge (1) von 0,7 bis 1,0, weiter bevorzugt von 0,8 bis 1,0, aufweist.

13. Piroctone-Olamine-Granulat mit einem $d_{50}$, bezogen auf eine Volumenverteilung, von 0,3 mm bis 6 mm und einem durchschnittlichen D/l-Verhältnis von Durchmesser (D) zu Länge (1) von 0,6 bis 1,0.

14. Piroctone-Olamine-Granulat nach Anspruch 13 mit einem $d_{50}$, bezogen auf eine Volumenverteilung, von 0,3 mm bis 2,5 mm, noch weiter bevorzugt 0,5 mm bis 2 mm.

**15.** Piroctone-Olamine-Granulat nach Anspruch 13 oder 14 mit einem durchschnittlichen D/l-Verhältnis von Durchmesser (D) zu Länge (1) von 0,7 bis 1,0, weiter bevorzugt von 0,8 bis 1,0.

**16.** Piroctone-Olamine-Granulat nach einem der Ansprüche 13 bis 15 mit einem $d_{90}$, bezogen auf eine Volumenverteilung, kleiner oder gleich 1,9 mm.

**17.** Piroctone-Olamine-Granulat nach einem der Ansprüche 13 bis 16 mit einem $d_{10}$, bezogen auf eine Volumenverteilung, größer oder gleich 0,4 mm.

**18.** Piroctone-Olamine-Granulat nach einem der Ansprüche 13 bis 17 mit einer Schüttdichte von 400 kg/m$^3$ bis 600 kg/m$^3$, vorzugsweise von 450 kg/m$^3$ bis 550 kg/m$^3$, weiter bevorzugt von 470 kg/m$^3$ bis 530 kg/m$^3$.

**Revendications**

**1.** Procédé pour la fourniture de granules de piroctone olamine possédant une plage cible de taille de particule, la plage cible de taille de particule étant une plage de $d_{50}$ cible, la plage de $d_{50}$ cible, sur la base d'une distribution volumique, étant de 0,3 mm à 6 mm, et les granules de piroctone olamine possédant un rapport D/l moyen, de diamètre (D) sur longueur (1) de 0,6 à 1,0, comprenant :

  a) la compression de cristaux de piroctone olamine pour former un compactat ;
  b) le broyage du compactat pour former des granules en vrac de piroctone olamine.

**2.** Procédé selon la revendication 1, comprenant en outre :
c) la séparation des granules de piroctone olamine possédant la plage cible de taille de particule à partir des granules en vrac.

**3.** Procédé selon la revendication 1 ou 2, la compression en a) étant réalisée à une pression de 25 bars à 200 bars, préférablement de 30 bars à 50 bars.

**4.** Procédé selon une quelconque revendication précédente, la pression appliquée dans l'étape de compression a) étant suffisante pour former un compactat possédant une densité de 980 kg/m$^3$ à 1 300 kg/m$^3$.

**5.** Procédé selon une quelconque revendication précédente, dans lequel en b) le broyage a lieu dans un broyeur à tamis, préférablement un broyeur à tamis rotatif ou un broyeur à tamis oscillant.

**6.** Procédé selon la revendication 5, la maille du broyeur à tamis rotatif ou du broyeur à tamis oscillant possédant une taille de maille de 10 mm ou moins, préférablement une taille de maille de 7 mm ou moins, plus préférablement une taille de maille de 4 mm ou moins, encore plus préférablement une taille de maille de 2 mm.

**7.** Procédé selon une quelconque revendication précédente, la plage de $d_{50}$ cible, sur la base d'une distribution volumique, étant de 0,3 mm à 2,5 mm, plus préférablement encore de 0,5 mm à 2 mm.

**8.** Procédé selon l'une quelconque des revendications 2 à 7, dans lequel en c) la séparation de granules de piroctone olamine dans la plage cible de taille de particule comprend :

  c1) une première séparation éventuelle réalisée sur les granules en vrac en utilisant un premier tamis possédant une taille de maille correspondant à la limite supérieure de la plage cible de taille de particule, la première séparation produisant un produit intermédiaire comprenant des granules de piroctone olamine qui sont passées à travers le premier tamis et un reste comprenant des granules grossiers de piroctone olamine qui ne sont pas passées ;
  c2) une deuxième séparation réalisée sur le produit intermédiaire ou, s'il n'y a pas de première séparation, alors sur les granules en vrac, en utilisant un deuxième tamis possédant une taille de maille correspondant à la limite inférieure de la plage cible de taille de particule, la deuxième séparation produisant des fines de piroctone olamine qui sont passées à travers le deuxième tamis et des granules de piroctone olamine, dans la plage cible de taille de particule, qui ne sont pas passées.

**9.** Procédé selon la revendication 8, comprenant en outre :

d) la recirculation des fines de piroctone olamine et l'ajout de celles-ci aux cristaux de piroctone olamine devant être compressés en a).

10. Procédé selon la revendication 8 ou 9, une première séparation ayant eu lieu, comprenant en outre :

e) le broyage du reste ;
f) la recirculation du reste broyé et l'ajout de celui-ci aux cristaux de piroctone olamine devant être compressés en a).

11. Procédé selon l'une quelconque des revendications précédentes, la compression en a) étant réalisée en l'absence de quelconques additifs.

12. Procédé selon l'une quelconque des revendications précédentes, les granules de piroctone olamine possédant un rapport D/l moyen, de diamètre (D) sur longueur (1) de 0,7 à 1,0, plus préférablement de 0,8 à 1,0.

13. Granules de piroctone olamine possédant un $d_{50}$, sur la base d'une distribution volumique, de 0,3 mm à 6 mm, et possédant un rapport D/l moyen, de diamètre (D) sur longueur (1) de 0,6 à 1,0.

14. Granules de piroctone olamine selon la revendication 13 possédant un $d_{50}$, sur la base d'une distribution volumique, de 0,3 mm à 2,5 mm, plus préférablement encore de 0,5 mm à 2 mm.

15. Granules de piroctone olamine selon la revendication 13 ou 14 possédant un rapport D/l moyen, de diamètre (D) sur longueur (1) de 0,7 à 1,0, plus préférablement de 0,8 à 1,0.

16. Granules de piroctone olamine selon l'une quelconque des revendications 13 à 15 possédant un $d_{90}$, sur la base d'une distribution volumique, inférieur ou égal à 1,9 mm.

17. Granules de piroctone olamine selon l'une quelconque des revendications 13 à 16 possédant un $d_{10}$, sur la base d'une distribution volumique, supérieur ou égal à 0,4 mm.

18. Granules de piroctone olamine selon l'une quelconque des revendications 13 à 17 possédant une densité en vrac de 400 kg/m$^3$ à 600 kg/m$^3$, préférablement de 450 kg/m$^3$ à 550 kg/m$^3$, plus préférablement de 470 kg/m$^3$ à 530 kg/m$^3$.

Figure 1

Figure 2

Figure 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 2234009 A1
- DE 1795270 A1

- US 4796812 A

**Non-patent literature cited in the description**

- **SCHULZE, D.** Pulver und Schüttgüter. Springer, 2009